# EUROPEAN PATENT SPECIFICATION

(11) **EP 0 546 639 B1**
(45) Date of publication and mention of the grant of the patent: **09.04.1997**
(21) Application number: 92203844.3
(22) Date of filing: 09.12.1992
(51) Int. Cl.: C07C 37/18, C07C 37/50, C07C 39/15, C07C 39/17, C07C 39/21

(54) **A process for the preparation of bisphenols**
Verfahren zur Herstellung von Bisphenolen
Procédé pour la préparation de bisphénols

(30) Priority: 11.12.1991 EP 91203275
(43) Date of publication of application: 16.06.1993
(73) Proprietor: SHELL INTERNATIONALE RESEARCH MAATSCHAPPIJ B.V., 2596 HR Den Haag (NL)
(72) Inventor: Meurs, Jan Hermen Hendrik, NL-1031 CM Amsterdam (NL); de Jong, Feike, NL-1031 CM Amsterdam (NL)

(56) References cited:
- EP-A- 0 013 436
- DE-A- 3 527 862
- FR-E- 90 426
- GB-A- 806 014
- US-A- 3 062 896
- CHEMICAL ABSTRACTS, vol. 72, no. 8, 23 February 1970, Columbus, Ohio, US; abstract no. 32610b, M.S. OGII ET AL. 'Synthesis of epoxy resins based on condensation products of phenol and bis(chloromethyl) derivatives of aromatic hydrocarbons.' page 34 ;column 2 ;

## Description

The invention relates to a process for the preparation of bisphenols having the chemical formula 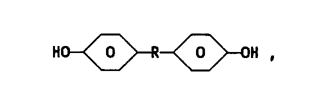 wherein R represents a divalent hydrocarbon radical and to novel intermediates and novel bisphenols per se.

The bisphenols are prepared in a process according to our invention in two steps, starting from 2,6-di-tert.butylphenol or 2,6-di-tert.amylphenol and a dihalide.

In the German Patent Application 3,527,862 (A1) (Offenlegungsschrift DE 3527862) is disclosed a process for the preparation of bis-hydroxylphenyl-n-alkanes wherein in a first step a phenol is reacted with an alkane dicarboxylic acid or an acid chloride therefrom in the presence of a fluorine containing organic sulphonic acid to a di-(4-hydroxyphenyl) substituted alkanedione and in a second step hydrogenating the dione. Moreover, the German Patent Application 3,527,862 further mentions aluminium chloride as an acidic catalyst in the first reaction step.

In U.S. Patent 3,062,896 there is disclosed the preparation of 1,4-bis(3,5-di-tert-alkyl-4-hydroxybenzyl)tetraalkylbenzene by reacting the alkali metal salt of 2,6-di-tert alkylphenol with 1,4-bis(halomethyl)tetraalkylbenzene, each of said alkyl groups having up to eight carbon atoms, under an inert atmosphere in an inert substantially anhydrous organic solvent.

In the European Patent application 0 013 436 there is disclosed a process for producing p,p'-biphenol which comprises heating a 4,4'-bis (substituted phenol) at elevated temperature below the decomposition temperature of p,p'-biphenol in the absence of a catalyst under an inert atmosphere.

It has now been found that when 2,6-di-tert.butylphenol or 2,6-di-tert.amylphenol is reacted with a dihalide, a basic catalyst performs well in the first reaction step. In a next step a debutylation or deamylation step gives the above-mentioned bisphenols. Advantages of the process are that strong acidic and corrosive media are avoided and that the reaction can be carried out in a more general way since the dihalide may contain a more versatile hydrocarbon radical in the starting product.

The invention is defined as relating to a process for the preparation of bisphenols of the chemical formula 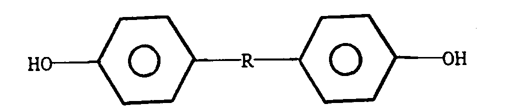 wherein R represents a divalent hydrocarbon radical which comprises reacting in a first step 2,6-di-tert.butylphenol or 2,6-di-tert.amyl phenol with a dihalide Hal-R-Hal, wherein Hal is a halogen atom and R has the above meaning, in the presence of a basic substance to an intermediate product of the chemical formula 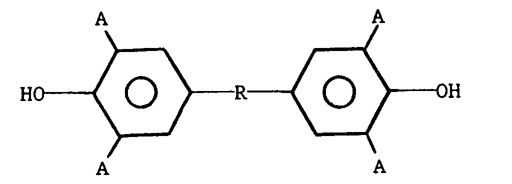 wherein A represents a tertiary butyl or a tertiary amyl group and in a second step debutylating or deamylating the intermediate product.

The divalent hydrocarbon radical may be considered to be derived from an alkane, a cycloalkane, an alkene, a cycloalkene or an aromatic hydrocarbon. The alkanes and alkenes may be linear or branched. Examples of divalent hydrocarbon radicals are linear alkylene groups having the formula -(CH₂)ₙ-, where n is an integer from 1 to 12, preferably from 4 to 12. Suitable are also the alkenes, linear or branched, such as where R represents 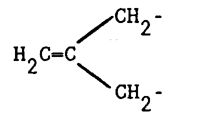 or

-H₂C-CH=CH-CH₂―

Other preferred R groups are

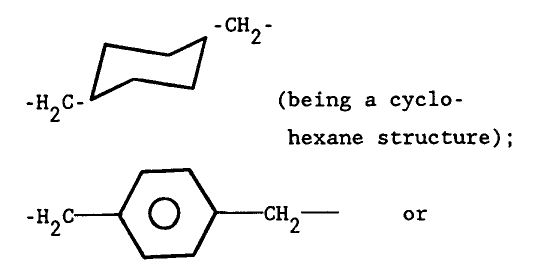

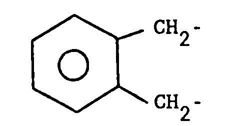

The corresponding dihalide Hal-R-Hal is preferably a dichloride or a dibromide.

As already indicated a basic substance is needed to carry out the reaction process. Preferably an alcoholate or a hydroxide is used. Alkali metals like sodium and potassium are the preferred ions.

Preferred basic substances used are sodium isopropylate, sodium ethoxylate, sodium hydroxide or sodium hydride. The corresponding potassium compounds can be used as well. They may be used when prepared in situ as well.

Suitable solvents are alcohols, such as ethanol, isopropanol or tert.butanol. Other suitable solvents may be other branched alcohols, such as pentanols, sec.butanol, ethers, benzene or toluene. The first reaction step to prepare the intermediate is preferably carried out at a temperature between O °C and 100 °C, although higher temperatures are not excluded.

The second step, the debutylating step is carried out at a temperature between 80 °C and 400 °C, depending on whether thermal cleavage is used alone or whether catalyst are used as well. In Example 1 three methods are disclosed. The obtained bisphenols are suitable as starting materials in the preparation of resins, polycarbonates and polyesters. A number of the intermediates and the bisphenols are novel compounds.

Novel are compounds of the chemical structure 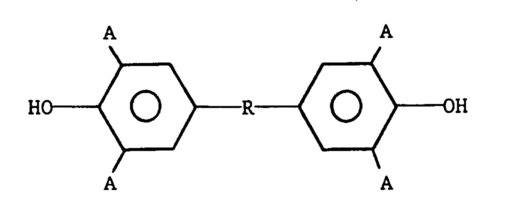 wherein A has the above significance and
R is: -(CH₂)ₙ- wherein n is 11 or 12;

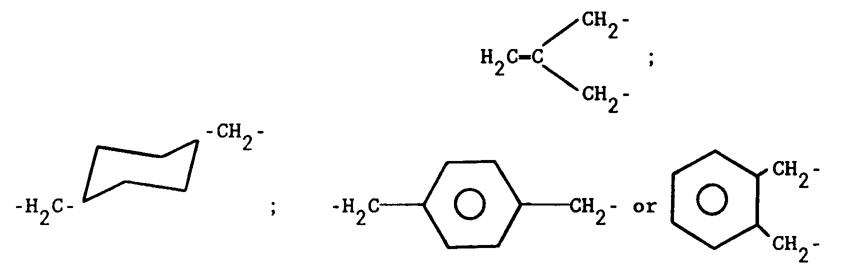

Novel are the bisphenols of the chemical structure 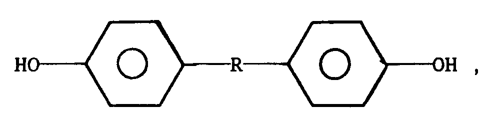 wherein R is: 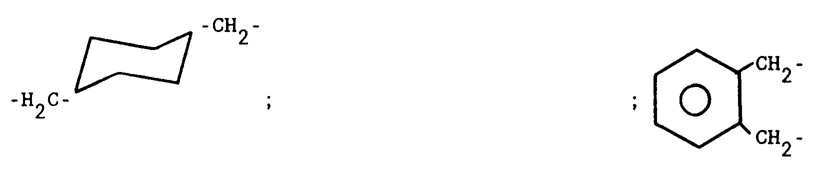 or -CH₂-CH=CH-CH₂-.

The first reaction step is finalized by adding an acid to the reaction mixture, preferably by adding an inorganic acid, e.g. HCl, generally in the form of an aqueous solution. Although this is not necessary, the acidification of the reaction mixture is preferred, since otherwise the formed alkali metal salt of the phenol in combination with the tertiary butyl groups acts as strong reducing agent.

Once the reaction mixture is acidified, in fact neutralized, the organic compounds are extracted with an organic solvent, e.g. a chlorinated hydrocarbon or an ether. The extract is washed with water to remove any inorganic salt left, and dried, e.g. over calcium dichloride.

The used organic solvent and the 2,6-di-tert.butylphenol (or 2,6-di-tert.amylphenol) are distilled off and the impure intermediate product can be further purified by crystallization. The intermediates are partly solid products, but may also be liquids, such as may be seen in Example 3.

### Example 1

A. To a stirred solution of 41.2 g (0.2 mol) of 2,6-di-tert.butylphenol (abbreviated: DTBP) in 100 ml isopropanol were added 4.6 g (O.2 mol) of sodium metal in 15 min under an inert atmosphere (N₂) at a temperature of 50 °C. After complete dissolution of the sodium 16.1 g (0.066 mol) of 1,6-dibromohexane were added in 1 hour.
   The resulting mixture was stirred for 16 h at 60 °C, whereafter the reaction mixture was cooled to 20 °C and hydrochloric acid (25 percent by weight of HCl in an aqueous solution) was added until the colour of the reaction mixture changed from green-blue into yellow. Then 200 ml dichloromethane were added and the total reaction mixture was extracted twice with 500 ml water.
   The organic phase (which had been extracted with water) was dried over calcium chloride, the solvent was subsequently evaporated and the excess of 2,6-di-tert.butylphenol was distilled off, while maintaining a bath temperature of 200 °C and a pressure of 133 Pa. Then 250 ml methanol were added to the residue and the resulting mixture heated to 70 °C. After cooling to 20 °C white crystals of 1,6-bis(4-hydroxy-3,5-di-tert.butylphenyl)hexane were formed and collected (13 gram), with a melting point of 147 °C and ¹H-NMR(CDCl₃, ppm)1-1.7(44H),2.5(4H),5.0(2H),6.99(4H).
B. Debutylation of the bisphenol was carried out by the following methods:
   1. Refluxing of the tert. butylphenol, prepared under A, as 10% solution in 47% HBr/acetic acid (75:15 vol) for 6 h.
   2. Thermal cleavage during 3 hours at 380 °C.
   3. Catalytic action of an aluminium ethoxide (1% by weight) during 3 hours at 280 °C under N₂.
      Debutylation by method 1 yielded 67% of the theory of 4,4'-hexamethylene bisphenol, also called 1,6-bis(4-hydroxyphenyl)hexane.
      Physical properties: melting point 146 °C
      - ¹H-NMR:: 1.0-1.5 (8H)
      2.32 (4H)
      4.60 (2H)
      6.62 (4H)
      6.84 (4H)

      Debutylation by method 2 gave 56% of the theoretical yield.
      Debutylation by method 3 gave 65% of the theoretical yield.

### Example 2

A. Reaction: 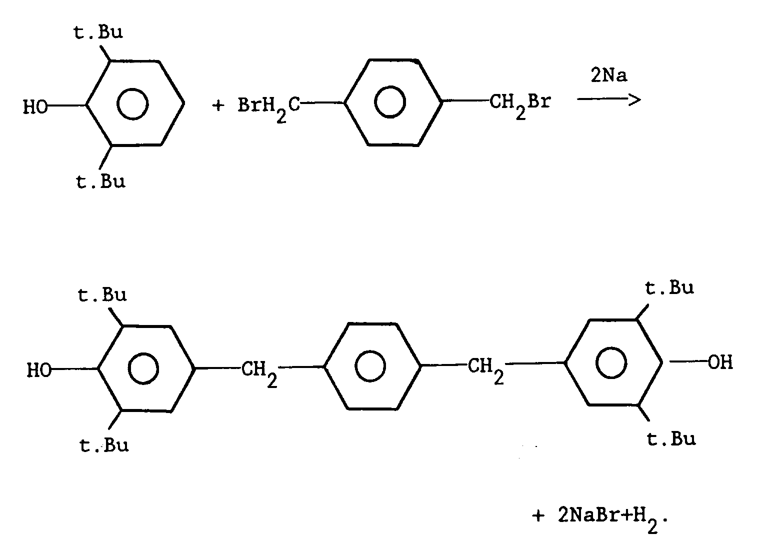
   Under N₂, sodium metal (0.6 mol, 13.8 g) was dissolved in a stirred solution of 2,6-di-tert.butyl phenol (1 mol, 206 g) in 500 ml ethanol. Then the dibromide (0.25 mol, 66 g) was added in about 45 min, while keeping the solution at 0 °C. The resulting mixture was stirred for 2 h at O °C, subsequently 16 h at 25 °C and further 2 h at 60 °C. The mixture was then cooled to 25 °C and acidified with a 33% aqueous HCl solution while the colour of the mixture turned from green into yellow.
   To the resulting reaction mixture were added 300 ml of diethylether and the mixture was 5 times extracted with 200 ml water. The ethereal solution was then dried over magnesium sulphate. Further, the solvent and the excess of 2,6-di-tert.butylphenol were distilled off. The obtained residue was recrystallized from methanol, yielding 59 g of product (68% of the theoretical yield) having a melting point of 152 °C and
   - ¹H-NMR:: 1.41 (36H)
   3.87 (4H)
   5.04 (2H)
   6.98 (4H)
   7.12 (4H).
B. The product of A (22 g) was debutylated by heating under N₂ for 1 hour at 380 °C in a fluidized sandbath until gas evolution stopped. The resulting debutylated product was recrystallized from chloroform (6.2 g = 50% of the theoretical yield) and had the chemical formula 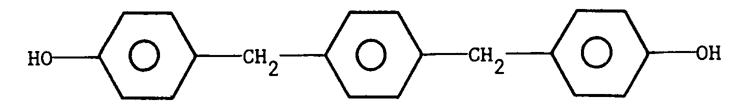
   Physical properties: melting point 187 °C
   - ¹H-NMR:: 3.0-3.5 (2H)
   3.79 (4H)
   6.76 (4H)
   6.95 (4H)
   7.02 (4H)

### Example 3

In an analogous manner as described in Example 1 were prepared:
1,12-bis(4-hydroxy-3,5-di-tert.butylphenyl)dodecane, liquid
1,11-bis(4-hydroxy-3,5-di-tert.butylphenyl)undecane, liquid
1,8-bis(4-hydroxy-3,5-di-tert.butylphenyl)octane, liquid
1,5-bis(4-hydroxy-3,5-di-tert.butylphenyl)pentane, liquid.

After the excess of DTBP and solvent had been distilled off at 200 °C under reduced pressure, methanol was added to the obtained residue. An oily substance was separated off.

The yields were 70%, 82%, 56% and 62% of the theoretical yield, calculated on the dibromo alkane, respectively.

The above-mentioned products were subsequently converted into the corresponding debutylated compounds by method B1 as in

### Example 1.

1,12-bis(4-hydroxyphenyl)dodecane m.p. 136 °C
   - ¹H-NMR:: 1.2 (16H)
   - (acetone): 1.6 ( 4H)
   2.5 ( 4H)
   7.0 ( 4H)
   6.7 ( 4H)
1,11-bis(4-hydroxyphenyl)undecane m.p. 101 °C
   - ¹H-NMR:: 1.3 (14H)
   - (acetone): 1.6 ( 4H)
   2.5 ( 4H)
   6.7 ( 4H)
   7.0 ( 4H)
1,8-bis(4-hydroxyphenyl)octane m.p. 134 °C
   - ¹H-NMR:: 1.4 (8H)
   - (acetone): 1.6 (4H)
   2.5 (4H)
   6.75 (4H)
   7.00 (4H)
   8.0 (2H)
1,5-bis(4-hydroxyphenyl)pentane m.p. 89 °C
   - ¹H-NMR:: 1.4 (2H)
   - (acetone): 1.6 (4H)
   2.4 (4H)
   6.8 (4H)
   7.0 (4H)
   and the corresponding dibenzoate had a melting point of 96 °C.

### Example 4

A. In an analogous manner as described in Example 2, but reacting as dihalo compound the 1,4-dibromo-butene-2 with DTBP, was prepared 1,4-bis(4-hydroxy-3,5-di-tert.butylphenyl)-butene-2. Physical properties: melting point 142 °C
   - ¹H-NMR:: 1.44 (36H)
   3.31 ( 4H)
   5.0 ( 2H)
   5.68 ( 2H)
   7.00 ( 4H)
   Also is formed the isomer 1,2-bis(4-hydroxy-3,5-di-tert.butylphenyl)-butene-1.
B. 4.92 g of the product were dissolved in 100 ml ethanol, to which solution was added 0.5 g of 5% Pd/C. During 16 hours a small stream of hydrogen is led through the reaction mixture, whereafter the 5% Pd/C is filtered off and the ethanol is distilled off.
   Yield of 1,4-bis(4-hydroxy-3,5-di-tert.butylphenyl)butane is 4.2 g (94% of the theory), melting point 124 °C.
   - ¹H-NMR:: 1.3-1.8 (40H)
   - (CDCl₃): 2.50 ( 4H)
   5.20 ( 2H)
   7.00 ( 4H)
C. The latter product was debutylated according to method B2 in Example 1 to 1,4-bis(4-hydroxyphenyl)butane, melting point 156 °C.
   - ¹H-NMR:: 1.25 (4H)
   - CDCl₃/pyridine d₅: 2.20 (4H)
   6.55 (4H)
   6.65 (4H)

### Example 5

A. In an analogous manner as described in Example 2 ortho-di-chloromethylbenzene was reacted with DTBP, and worked up in the same manner. A product of the chemical formula 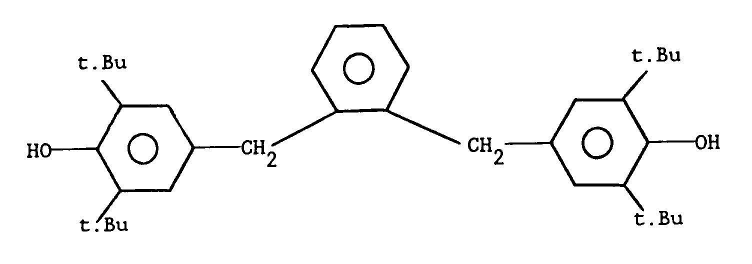 with a melting point of 127 °C and
   - ¹H-NMR:: 1.41 (36H)
   3.95 ( 4H)
   5.03 ( 2H)
   6.94 ( 4H)
   7.0-7.25 ( 4H)
   in a yield of 57% of the theory was obtained.
B. The latter compound, debutylated according to method B2 in Example 1 gave a product of the chemical formula 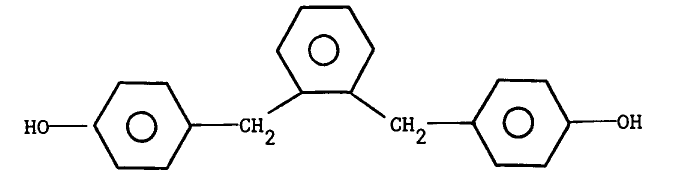 in a yield of 78% (of the theory), a melting point of 141 °C and
   - ¹H-NMR:: 3.86 (4H)
   6.81 (4H)
   6.93 (4H)
   7.13 (4H)
   8.17 (2H)
C. Instead of ortho-dichloromethylbenzene the corresponding ortho-dibromomethylbenzene was used with practically the same results.

### Example 6

A. In an analogous manner as described in Example 2 para-di-bromomethylcyclohexane was reacted with DTBP. A product of the chemical formula 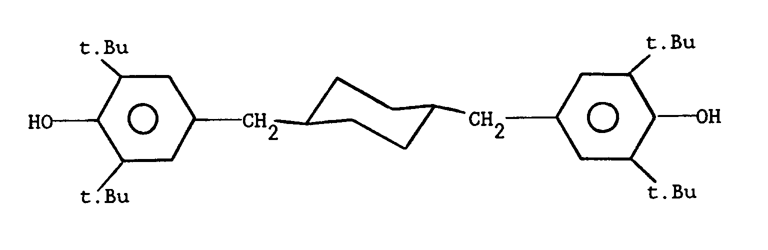 with a melting point of 202 °C and
   - ¹H-NMR:: 1.48 (36H)
   0.95-1.9 (10H)
   2.43 ( 4H)
   5.05 ( 2H)
   6.96 ( 4H)
   in a yield of 46% of the theory was obtained.
B. The latter product was debutylated according to method B3 of Example 1, to a product of the chemical formula 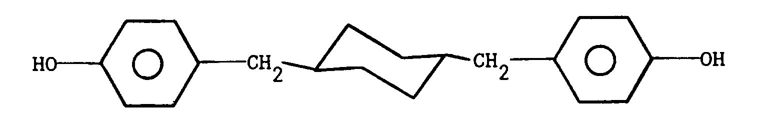 in a yield of 67% of the theory, with a melting point of 226 °C and
   - ¹H-NMR:: 0.90 (4H)
   1.40 (2H)
   1.70 (4H)
   4.00 (2H)
   6.87 (4H)
   6.99 (4H)

### Example 7

In an analogous manner as described in Example 2 methalyldichloride 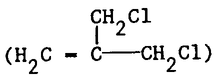 was reacted with DTBP.
A product of the chemical formula 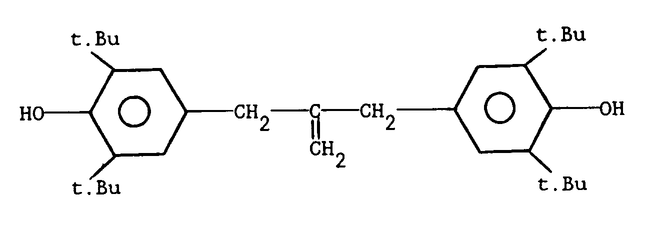 with a melting point of 146 °C and
- ¹H-NMR:: 1.46 (36H) +
3.22 ( 4H)
4.80 ( 2H)
5.05 ( 2H)
6.96 ( 4H)
in a yield of 58% (of the theory) was obtained. The product was hydrogenated and subsequently debutylated.

### Example 8

To a stirred solution of 206 g (1 mol) of 2,6-di-tert.butylphenol in 500 ml isopropanol were added 40 g (1 mol) of sodium hydroxide in a nitrogen atmosphere. After complete dissolution of the sodium hydroxide 81.3 g (0.33 mol) of 1,6-dibromohexane were added under stirring during 60 min. The resulting mixture was further stirred for 16 hours at 60 °C.

After working up of the reaction mixture in the same way as in Example 1, under A, 65 g (yield 40% of the theory) of 1,6-bis(4-hydroxy-3,5-di-tert.butylphenyl)hexane, melting point 147 °C, were obtained.

## Claims

1. A process for the preparation of bisphenols of the chemical formula 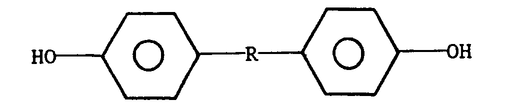 wherein R represents a divalent hydrocarbon radical, which comprises in a first step reacting 2,6-di-tert.butylphenol or 2,6-di-tert.amylphenol with a dihalide Hal―R―Hal, wherein Hal is a halogen atom and R has the above meaning, in the presence of a basic substance to an intermediate product of the chemical formula 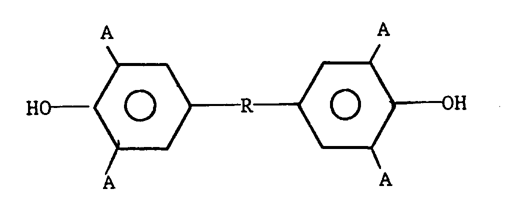 wherein A represents a tertiary butyl or a tertiary amyl group and in a second step debutylating or de-amylating the intermediate product.

2. A process as claimed in claim 1 wherein the divalent hydrocarbon radical R is derived from an alkane, a cycloalkane, an alkene, a cycloalkene or an aromatic hydrocarbon.

3. A process as claimed in claim 1 or 2 wherein the divalent hydrocarbon radical R is ―(CH₂)ₙ― wherein n is an integer from 4 to 12;

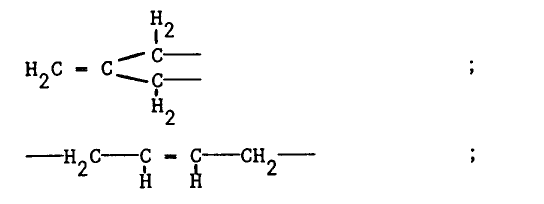

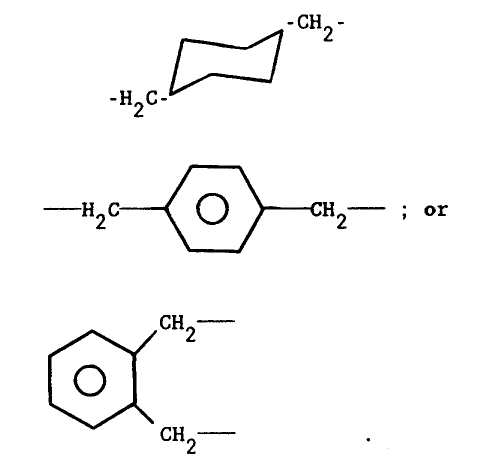

4. A process as claimed in one or more of the claims 1-3 wherein the basic substance is an alcoholate or a hydroxide.

5. A process as claimed in claim 5 wherein the basic substance is sodium isopropylate, sodium ethoxylate, sodium hydroxide or sodium hydride.

6. A process as claimed in one or more of the claims 1-5 wherein an alcohol is used as a solvent.

7. A process as claimed in one or more of the claims 1-5 wherein isopropanol, ethanol or tert.butanol is used.

8. A process as claimed in one or more of the claims 1-7 wherein the first reaction step is carried out at a temperature between 0 °C and 100 °C and the second reaction step at a temperature between 80 °C and 400 °C.

## Patentansprüche

1. Verfahren zur Herstellung von Bisphenolen der chemischen Formel 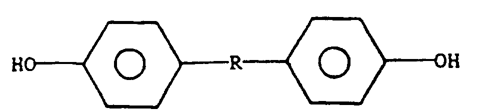 worin R einen zweiwertigen Kohlenwasserstoffrest bedeutet, bei dem man in einem ersten Schritt 2,6-Di-tert.-butylphenol bzw. 2,6-Di-tert.-amylphenol mit einem Dihalogenid Hal―R―Hal, worin Hal ein Halogenatom bedeutet und R die obengenannte Bedeutung besitzt, in Gegenwart einer basischen Substanz zu einem Zwischenprodukt der chemischen Formel 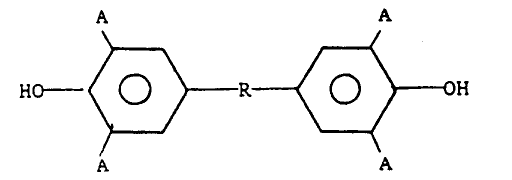 worin A eine tertiäre Butyl- bzw. eine tertiäre Amylgruppe bedeutet, umsetzt und in einem zweiten Schritt das Zwischenprodukt debutyliert bzw. deamyliert.

2. Verfahren nach Anspruch 1, bei dem sich der zweiwertige Kohlenwasserstoffrest R von einem Alkan, einem Cycloalkan, einem Alken, einem Cycloalken oder einem aromatischen Kohlenwasserstoff ableitet.

3. Verfahren nach Anspruch 1 oder 2, bei dem es sich bei dem zweiwertigen Kohlenwasserstoffrest R um -(CH₂)ₙ₋, worin n eine ganze Zahl von 4 bis 12 bedeutet; 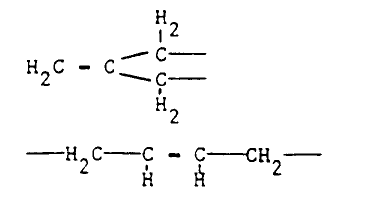 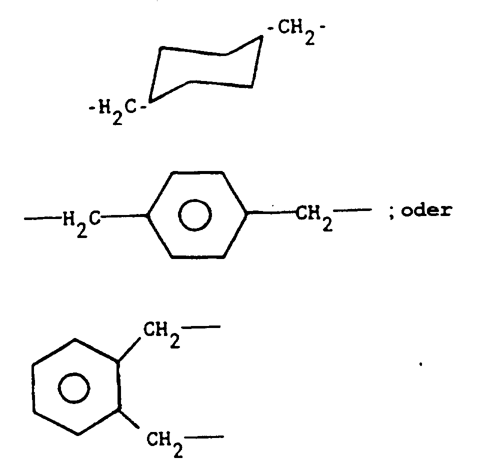 handelt.

4. Verfahren nach einem oder mehreren der Ansprüche 1 bis 3, bei dem man als basische Substanz ein Alkoholat oder ein Hydroxid einsetzt.

5. Verfahren nach Anspruch 4, bei dem man als basische Substanz Natriumisopropylat, Natriumethoxylat, Natriumhydroxid oder Natriumhydrid einsetzt.

6. Verfahren nach einem oder mehreren der Ansprüche 1-5, bei dem man als Lösungsmittel einen Alkohol einsetzt.

7. Verfahren nach einem oder mehreren der Ansprüche 1-5, bei dem man Isopropanol, Ethanol oder tert.-Butanol einsetzt.

8. Verfahren nach einem oder mehreren der Ansprüche 1-7, bei dem man den ersten Reaktionsschritt bei einer Temperatur von 0°C bis 100°C und den zweiten Reaktionsschritt bei einer Temperatur von 80°C bis 400°C durchführt.

## Revendications

1. Procédé de préparation de bisphénols de formule chimique 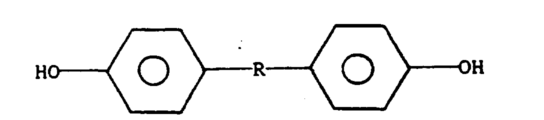 où R représente un radical hydrocarboné bivalent, qui comprend dans une première étape, la réaction de 2,6-di-t-butylphénol ou 2,6-di-t-amylphénol avec un dihalogénure, Hal-R-Hal, où Hal est un atome d'halogène et R a la signification ci-dessus, en présence d'une substance basique, en un produit intermédiaire de formule chimique 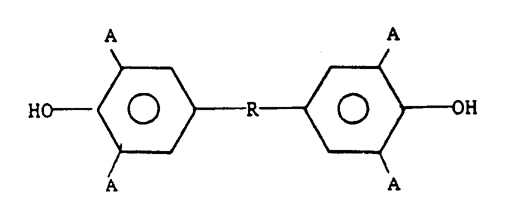 où A représente un radical t-butyle ou t-amyle et dans une deuxième étape, débutylation ou déamylation du produit intermédiaire.

2. Procédé suivant la revendication 1, dans lequel le radical hydrocarboné bivalent R dérive d'un alcane, d'un cycloalcane, d'un alcène, d'un cycloalcène ou d'un hydrocarbure aromatique.

3. Procédé suivant la revendication 1 ou 2, dans lequel le radical hydrocarboné bivalent R est -(CH₂)ₙ-, où n est un entier de 4 à 12;

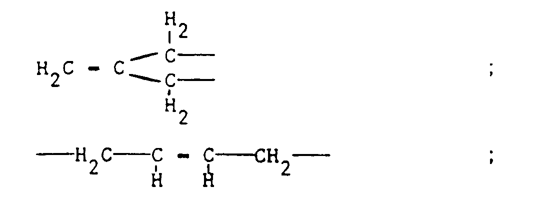

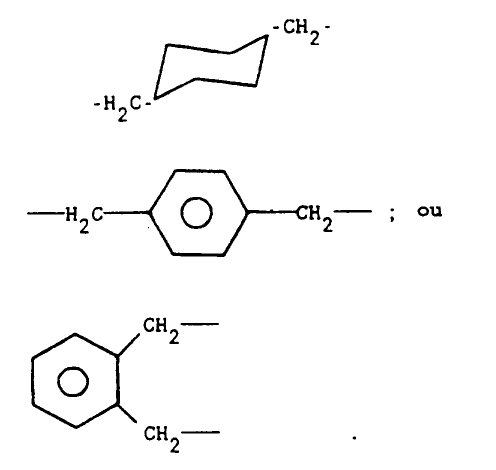

4. Procédé suivant l'une ou plusieurs des revendications 1 à 3, dans lequel la substance basique est un alcoolate ou un hydroxyde.

5. Procédé suivant la revendication 4, dans lequel la substance basique est de l'isopropylate de sodium, de l'éthoxylate de sodium, de l'hydroxyde de sodium ou de l'hydrure de sodium.

6. Procédé suivant l'une ou plusieurs des revendications 1 à 5, dans lequel on utilise un alcool comme solvant.

7. Procédé suivant l'une ou plusieurs des revendications 1 à 5, dans lequel on utilise de l'isopropanol, de l'éthanol ou du t-butanol.

8. Procédé suivant l'une ou plusieurs des revendications 1 à 7, dans lequel on réalise la réaction de la première étape à une température comprise entre 0°C et 100°C et la réaction de la deuxième étape, à une température comprise entre 80°C et 400°C.
